# EUROPÄISCHE PATENTSCHRIFT

(11) **EP 1 392 191 B1**
(45) Veröffentlichungstag und Bekanntmachung des Hinweises auf die Patenterteilung: **16.04.2008**
(21) Anmeldenummer: 02700142.9
(22) Anmeldetag: 08.02.2002
(51) Int. Cl.: A61B 17/80

(54) **Implantatplatte, Verfahren und Anlage zu deren Herstellung**
Implant plate, and process and plant for manufacture thereof
Plaque à implanter, et procédé et installation pour sa production

(30) Priorität: 16.02.2001 DE 10107369
(43) Veröffentlichungstag der Anmeldung: 03.03.2004
(73) Patentinhaber: Wiedemann, Ernst, 80997 München (DE); Zeiler, Claudius, 81375 München (DE)
(72) Erfinder: Wiedemann, Ernst, 80997 München (DE); Zeiler, Claudius, 81375 München (DE)
(74) Vertreter: Lohr, Georg
(86) Internationale Anmeldenummer: PCT/DE2002/000025
(87) Internationale Veröffentlichungsnummer: WO 2002/065928

(56) Entgegenhaltungen:
- EP-A- 0 791 338
- EP-A- 0 934 731
- WO-A-98/09578
- DE-A- 19 750 493
- "PHILOS LCP Locking Compression Plate" Januar 2001 (2001-01) , STRATEC MEDICAL , OBERDORF, SCHWEIZ XP002205191 in der Anmeldung erwähnt das ganze Dokument

## Beschreibung

Die Erfindung betrifft eine Implantatplatte, ein Verfahren und eine Anlage zur halb- oder vollautomatischen Herstellung von Implantatplatten.

Die Erfindung bezieht sich demzufolge auf eine mindestens einteilige interne Humerus-Platte. Außerdem umfaßt die Erfindung ein Verfahren zur halb- oder vollautomatischen Herstellung von Implantatplatten sowie eine Anlage zur Durchführung des Verfahrens. Mit der erfindungsgemäßen Implantatplatte wird eine wesentlich verbesserte Stabilisierung einer Fraktur des Oberarmkopfes und/oder einer Fraktur eines proximalen Oberarms erreicht.

Zum allgemeinen Stand der Technik gehören metallische Implantatplatten, die über den Längs- und den Querschnitt eine weitgehend konstante Dicke bzw. Stärke aufweisen. Mit den Implantatplatten werden die Knochenfrakturen unter Verwendung von Knochenschrauben lagemäßig fixiert und während des Heilungsprozesses zusammengehalten. Aus dem Prospekt "PHILOS-Proximal Humerus Internal LOcked System" Nov. 00 / EMA / Docseries V1, Fa. Stratec / Fa. Mathys, ist eine spezielle, schuhlöffelförmige Implantatplatte zur Stabilisierung einer proximalen Humerus-Fraktur bekannt. Der Oberbegriff des Anspruchs 1 basiert auf dieser Offenbarung. Über den mittleren Plattenabschnitt sind Bohrungen für Knochenschrauben vorgesehen, die einerseits in den Oberarmkopf mit den spongiösen Tubercula und andererseits in den proximalen Schaftbereich des Humerus gerichtet sind. Diese schuhlöffelförmigen Implantatplatten sind im kopfnahen Bereich des Oberarms verbreitert und im Schaftbereich des Humerus verschmälert ausgeführt. Der kopfnahe Bereich der Implantatplatte weist mindestens vier und der Schaftbereich mindestens drei Bohrungen auf Außerdem besitzt der kopfnahe Bereich der Implantatplatte mindestens sechs randnahe Bohrungen zur Aufnahme von metallischem Drahtmaterial oder resorbierbarem und nichtresorbierbarem Fadenmaterial, mit dem die Frakturteile des Kopfes zusammengehalten werden können. Aus der DE 197 50 493 A1 ist ein Implantat zur Stabilisierung einer Humeruskopf-Fraktur mittels einer Schraube zur Verwendung in der Chirurgie bekannt. Obwohl dieses Implantat in der DE 197 50 493 A1 wiederholt auch als Platte bezeichnet wird, liegt dem konstruktiven Aufbau im wesentlichen ein massiver Vierkantstab zugrunde, dessen Schaftbereich eine verringerte Materialstärke und dessen kopfnaher Bereich eine demgegenüber im wesentlichen verdoppelte Materialstärke aufweist. Der kopfnahe Bereich des Vierkantstabes ist im wesentlichen konkav zum Humeruskopf ausgeführt. Die Herstellung der Basisraumform des Implantats erfordert kostenintensive Verformungs-maßnahmen durch Schmieden, Pressen und/oder spanabhebende Bearbeitung. Dieses Implantat weist im kopfnahen Bereich zwei Führungen für Knochenschrauben auf, mit denen diese Schrauben zur Fixierung der Fraktur stabil in einem Winkel zur Platte gehalten werden. Vorzugsweise werden die Schrauben mit einer Einspannvorrichtung stabil gegen Drehungen um den Schraubenschaft und Verschiebungen in Richtung des Schraubenschaftes lösbar fixiert. Hierzu eignet sich eine spezielle Schraube mit einem Gewindeaußendurchmesser, der größer ist als der angrenzende Schaftdurchmesser. Um eine sichere Führung zu gewährleisten, ist der Schaftdurchmesser im Bereich des Schraubenkopfes vergrößert. Außerdem besitzt das Implantat Bohrungen für Schaftschrauben, die im wesentlichen senkrecht zur Längsachse des stabförmigen Implantats ausgerichtet sind. Am oberen freien Ende und zwischen den beiden Oberarmkopfschrauben des Implantatbereiches sind parallel zur Ober- und Unterseite des stabförmigen Implantats verlaufende Bohrungen vorgesehen, die der Aufnahme von Drahtcerclagen, resorbierbarem oder nichtresorbierbarem Nahtmaterial zur Refixation ausgerissener Tubercula dienen.

Die spezielle, schuhlöffelförmige Implantatplatte zur Stabilisierung einer Oberarmkopf-Fraktur, wie sie aus dem Prospekt "PHILOS-Proximal Humerus Internal LOcked System" Nov. 00 / EMA / Docseries V1, Fa. Stratec / F. Mathys, bekannt ist, besitzt zwar im kopfseitigen Abschnitt randnahe Bohrungen für die Aufnahme von Drahtcerclagen oder resorbierbarem oder nichtresorbierbarem Nahtmaterial, jedoch erschwert diese Konstruktion das Einfädeln dieser Befestigungselemente und deren Plattenrandumführung, so daß wegen der geringen Führungslänge das Nahtmaterial beschädigt werden kann und die Drahtcerclage verkanten kann. In beiden Fällen kann das Befestigungselement reißen. Außerdem verhindern die Befestigungselemente durchmesserbedingt zunächst eine flächige Plattenanlage an die Knochenoberfläche und erschweren ein nachträgliches Einfädeln. Das vierkantförmige Implantat, welches aus DE 197 50 493 A1 bekannt ist, weist zwar in seinem massiven, kopfseitigen Abschnitt zwei hierauf verteilte Bohrungen auf, die parallel zur Ober- und Unterseite des Implantats verlaufen, jedoch ist diese Ausführungsform auf eine Implantatplatte nicht ohne weiteres übertragbar. Die beiden Bohrungen sind danach nur möglich, weil der massive, kopfnahe Abschnitt des Implantats eine ausreichende Materialverstärkung im Vergleich zum schaftnahen Abschnitt aufweist. Im übrigen ist dieses Implantat wegen seiner extrem massiven Ausführung für den chirurgischen Gebrauch nicht ohne weiteres einsetzbar, weil die massiven proximalen Oberarmkopfschrauben durch erforderliches Vorbohren zusätzliche Knochenverluste verursachen. Weiterhin ist eine derart massive Ausführungsform aufgrund der relativ geringen Belastung an dieser oberen Extremität nicht erforderlich und störend für die umgebenden Weichteile.

Der Erfindung liegt die Aufgabe zugrunde, eine Implantatplatte mit wesentlich verbesserter Stabilisierung einer Fraktur eines Oberarmkopfes und/oder einer Fraktur des proximalen Oberarms zu schaffen. Außerdem soll ein Verfahren zur halb- oder vollautomatischen Herstellung der Implantatplatten und eine Anlage zur Durchführung des Verfahrens geschaffen werden. Die Erfindung soll auch eine Verwendung der Implantatplatte in der Chirurgie und/oder Orthopädie ermöglichen.

Diese Aufgabe wird mit dem Patentanspruch 1, dessen Merkmale eine Implantatplatte definieren, mit dem Patentanspruch 10, dessen Merkmale ein Verfahren zur Herstellung der erfindungsgemäßen Implantatplatte betreffen und mit dem Patentanspruch 13, dessen Merkmale eine Anlage zur Durchführung des Verfahrens umfassen, gelöst. Die jeweils hierauf rückbezogenen Unteransprüche gestalten die erfindungsgemäße Implantatplatte konstruktiv, das erfindungsgemäße Verfahren zur Herstellung der Implantatplatte technologisch und die erfindungsgemäße Anlage anordnungs- und maschinentechnisch weiter aus.

Das der Erfindung zugrunde liegende Lösungsprinzip besteht im wesentlichen darin, daß eine mit Bohrungen und/oder Langlochbohrungen für Knochenschrauben ausgestattete, löffelförmige Implantatplatte zur Stabilisierung einer Fraktur eines Oberarmkopfes und/oder eines Oberarms bereitgestellt wird, die mindestens eine erhabene Aufnahme für flexible Befestigungselemente aufweist, welche auf der dem Knochen abgewandten Seite und auf der Längsachse am oberen Rand des löffelförmigen Abschnitts angeordnet ist. Nach einer Ausführungsform kann auf der dem Knochen abgewandten Seite der lmplantatplatte zusätzlich mindestens je eine Aufnahme am Längsrand angeordnet sein. Jede Aufnahme besitzt eine zum jeweiligen Plattenrand im wesentlichen parallel verlaufende Aussparung. Die Aussparungen sind vorzugsweise ösen-, rundhaken- oder rohrförmig ausgeführt, wobei leicht gekrümmte und relativ lange Führungswege innerhalb der Aussparungen das Einfädeln, Durchführen und Zusammenziehen der verwendeten flexible Befestigungselemente, die um die Fraktur geschlungen werden, wesentlich erleichtern und verbessern. Die Ein- und Austrittsränder der Aussparungen müssen gebrochen, gerundet und/oder geglättet sein, damit die flexiblen Befestigungselemente nicht beschädigt oder zerstört werden. Jede Implantatplatte kann eine auf der Längsachse liegende, vom oberen Rand des löffelförmigen Abschnitts der Implantatplatte ausgehende Klinge aufweisen, die im abgewinkelten Zustand in den Humeruskopf eingetrieben wird. Die Klinge besitzt mindestens eine Bohrung mit jeweils mindestens einem Gewindegang, in welche die von dem kopfseitigen Abschnitt bzw. Kopfabschnitt der Implantatplatte schräg nach oben gerichtete Oberarmkopfschraube eingeschraubt wird. Die Implantatplatte weist zur Durchführung mindestens einer Oberarmkopfschraube im kopf und schaftseitigen Abschnitt jeweils mindestens eine Bohrung und /oder Langlochbohrung auf. Durch das Einbringen der Oberarmkopfschrauben von der Implantatplatte zur abgewinkelten Klinge wird eine stabile Winkelverbindung geschaffen, wodurch der Halt der Klinge im Humeruskopf und der Implantatplatte am Oberarmkopf und dem proximalen Oberarm verbessert wird. Die auf der dem Knochen abgewandten Seite der Implantatplatte randnah angeordneten Aufnahmen verbessern entscheidend das Einfädeln, Führen und Zusammenziehen des flexiblen Befestigungselements, mit welchem die Fraktur des Oberarmkopfes und/oder des proximalen Oberarms zusätzlich stabilisiert wird. Dieser konstruktive Aufbau der Implantatplatte leistet somit das Einführen, Ein- und Durchfädeln sowie Festziehen der flexiblen Befestigungselemente auch nach dem Befestigen der Platte am Knochen.

Zum erfindungsgemäßen Lösungsprinzip gehört auch ein Verfahren, nach welchem aus metallischem Flachband bzw. Bandmaterial, bestehend beispielsweise aus Implantatstahl, Titan oder Titanlegierungen udgl. die Implantatplatten programmgesteuert halb- oder vollautomatisch sowie halb- oder vollkontinuierlich hergestellt werden. Das Flachband, das mit der erforderlichen Dimensionierung entweder abgelängt in einem Stapelmagazin oder als Coil bereitgestellt wird, kann nach einer Ausführungsform zunächst kaltverformt werden, um auf der vorbestimmten knochenseitigen Anlagefläche der Implantatplatte beispielsweise Noppen, unterbrochene Rillen, Rauten udgl. zu erzeugen, damit die Versorgung von Knochenhaut und Knochen beim späteren Einsatz der Implantatplatte erhalten bleibt. Das Flachband kann auch durch vorausgehendes Warm- und/oder Kaltwalzen entsprechend oberflächenprofiliert als Ausgangsmaterial für das erfindungsgemäße Verfahren bereitgestellt werden. Daraufhin wird das Flachband in vorher festgelegter Anordnung zerspanend udgl. bearbeitet, um Aussparungen wie Bohrungen, Öffnungen udgl. mit relativ großem Durchmesser für die Knochenschrauben und Aussparungen wie Bohrungen, Öffnungen udgl. mit relativ kleinem Durchmesser in den aus dem Flachbandmaterial zu formenden Aufnahmen für flexible Befestigungselemente zu erzeugen. Gleichzeitig oder anschließend werden die für die Bildung der Aufnahmen und ggf. der Klinge erforderlichen randnahen Formschnitte wie Parallelschlitze, Schrägschnitte, Teilausschnitte udgl. in dem Flachband beispielsweise durch Schneiden, Stanzen, Lasern, Zerspanen, Erodieren udgl. ausgeführt. Ebenfalls gleichzeitig oder anschließend werden in Bezug auf den späteren Einsatz der Implantatplatte die für die Bildung der randnahen Aufnahmen vorbereiteten Bereiche des Flachbandes in Richtung der dem Knochen abgewandten Seite kaltverformt, d.h. tiefgezogen, gebogen, gerollt, abgewinkelt udgl., so daß sich die Aufnahmen für die flexiblen Befestigungselemente auf der dem Knochen abgewandten Seite der Implantatplatte erhaben, aber mit geringer Höhe abbilden. Die in den Aufnahmen vorher hergestellten Aussparungen wie Bohrungen, Öffnungen udgl. sind dann im wesentlichen parallel zum Verlauf des Plattenrandes sowie der Ober- und Unterseite der noch fertigzustellenden Implantatplatte randnah positioniert. Die Kaltverformung kann auch dazu eingesetzt werden, daß quer zur Längsachse verlaufende, beabstandete Materialschwächungsbereiche zur späteren Nutzung als Filmscharniere oder Trennungsstellen zur Bildung von Verhakungsscharnieren gebildet und die Kanten am freien Ende der Klinge geschärft werden. Danach kann das soweit vorbereitete Flachband zu einer flachen, den Klingenbereich im wesentlichen nicht erfassenden Längsrinne kaltumgeformt werden, um beim späteren Einsatz der Implantatplatte deren Anlage an den Knochen zu optimieren. Anschließend werden aus dem bombierten Bandmaterial die Implantatplattenrohlinge mit der löffelförmigen Außenkontur durch Materialabtrennungen, beispielsweise durch Stanzen, Lasern, Schneiden, Zerspanen udgl., hergestellt und dabei gleichzeitig vereinzelt.

Nach einer verfahrenstechnischen Ausführungsform wird ein Flachband, das breiter ist als die Breite des löffelförmigen Abschnitts der Implantatplatte, verwendet, um aus dem überstehenden Plattenrandbereich breite oder schmale rechteckförmige Stege mit ausreichender Länge zu stanzen, lasern, schneiden udgl.. Durch Einrollen dieser Stege in Richtung auf die dem Knochen abgewandte Oberseite der Implantatplatte werden rohr-, ösen- oder rundhakenförmige Aufnahmen für das Durchziehen von flexiblen Befestigungselementen mit geringer Höhe gebildet. Die Vereinzelung des soweit vorbereiteten Flachbandes zu Implantatplattenrohlingen erfolgt ebenfalls durch entsprechende Materialabtrennung. Die vereinzelten Implantatplattenrohlinge erhalten anschließend durch Kaltumformung das vorbestimmte flache Rinnenprofil.

Nach einer bevorzugten verfahrenstechnischen Ausführungsform können anstelle der verfahrenstechnischen Vorbereitungsmaßnahmen zur Bildung der Aufnahmen aus dem Flachbandmaterial extern vorgefertigte Aufnahmen für die flexiblen Befestigungselemente in Gestalt von Rohrabschnitten, Rundhaken, Ösen udgl. mit gering auftragender Höhe und auf den Durchmesser der flexiblen Befestigungselemente abgestimmten Öffnungsquerschnitt randnah auf der dem Knochen abgewandten Oberfläche der vereinzelten Implantatplattenrohlinge vor oder nach deren Bombierung, d.h. flachen Rinnenformgebung, durch Kaltumformen aufgeschweißt, preßgeschweißt, gelötet, geschraubt udgl. befestigt werden.

Die mit Aufnahmen für flexible Befestigungselemente ausgestatteten Implantatplattenrohlinge erreichen nach dem Entgraten, Kantenrunden und Säubern den Fertigungsendzustand. Nach einer verfahrenstechnischen Ausführungsform können die Implantatplatten mit galvanischen, elektrochemischen, vakuumtechnischen wie CVD-Methode (Chemical Vapour Deposition) oberflächenveredelt werden. Die Implantatplatten werden sterilisiert und entsprechend verpackt der chirurgischen Verwendung zugeführt.

Die zur Erfindung gehörende Anlage ist linear und modular aufgebaut und umfaßt im wesentlichen programmgesteuerte, halb- oder vollautomatisch und getaktet arbeitende Fertigungsmaschinen oder -automaten, mit denen das Flachband zur Bildung der Aufnahmen für flexible Befestigungselemente bearbeitet sowie die Implantatplatte ggf. einschließlich der Klingen hergestellt werden.

Nach einer anlagetechnischen Ausführungsform können die einzelnen Vorrichtungen in linearer Ausrichtung, gegenüberliegend oder mäanderförmig sowie auf einem Karussell, Drehkreuz oder Doppelstern funktionsmäßig zusammenarbeitend angeordnet sein.

Nach der Erfindung werden die Implantatplatten sowie die nach dem Verfahren ggf. unter Einsatz der Anlage unmittelbar hergestellten Implantatplatten für die Stabilisierung von Frakturen des Oberarmkopfes und des proximalen Oberarms verwendet.

Die Erfindung wird in den Zeichnungen näher erläutert.

Figur 1a zeigt in Frontansicht mit eingetragenem Längsschnitt A-A eine löffelförmig gestaltete Implantatplatte zur zusätzlichen Stabilisierung der Fraktur des Oberarmkopfes und/oder des proximalen Oberarms mit Bohrungen und/oder Langlochbohrungen für Knochenschrauben im löffel- und schaftseitigen Abschnitt sowie stegförmige Aufnahmen für flexible Befestigungselemente im randnahen Bereich des kopfseitigen Abschnitts bzw. Löffelabschnitts der Implantatplatte.

Figur 1b zeigt in Seitenansicht die Anordnung der Implantatplatte an dem Oberarmkopf und dem proximalen Oberarm nach Figur 1 a gemäß Längsschnitt A-A.

Figur 1c zeigt in Draufsicht die Implantatplatte.

Figur 2 zeigt eine vergrößerte stegförmige Aufnahme in Front- und Seitenansicht sowie Draufsicht nach Figur 1a bis 1c mit Schnitt B-B in der Seitenansicht.

Figur 3a zeigt in Frontansicht eine löffelförmige Implantatplatte mit dem verbreiterten kopfseitigen Abschnitt bzw. Löffelabschnitt und dem schmäleren schaftseitigen Abschnitt bzw. Schaftabschnitt mit verteilt angeordneten Bohrungen und/oder Langlochbohrungen für Knochenschrauben und rohrförmigen Aufnahmen für flexible Befestigungselemente im randnahen Bereich des kopfseitigen Abschnitts sowie einer vom oberen Ende des kopfseitigen Abschnitts ausgehenden Klinge zur zusätzlichen Stabilisierung der Fraktur des Oberarmkopfes und des proximalen Oberarms.

Figur 3b zeigt in Seitenansicht die Anordnung der löffelförmigen Implantatplatte an dem Oberarmkopf und dem proximalen Oberarm mit eingetriebener Klinge sowie den rohrförmigen Aufnahmen nach Figur 3a.

Figur 4 zeigt in Front- und Seitenansicht sowie in Draufsicht eine rohrförmige Aufnahme im randnahen Bereich des kopfseitigen Abschnitts nach den Figuren 3a und 3b.

Figur 5 zeigt in Frontansicht eine Implantatplatte auf der Basis der Figuren 1a und 3a mit kreisförmig dargestellten, variablen Platzhalterbereichen für Aufnahmen im randnahen Bereich des kopfseitigen Abschnitts zur zusätzlichen Stabilisierung der Fraktur des Oberarmkopfes und des proximalen Oberarms.

Figur 6 zeigt in Front- und Seitenansicht sowie in Draufsicht eine aus einem tiefgezogenen, randnahen Schlitz geschaffene, ösenförmige Aufnahme für ein flexibles Befestigungselement in einer variablen Anordnung nach Figur 5.

Figur 7 zeigt in Front- und Seitenansicht sowie in Draufsicht eine aus einer randnahen Falte geschaffene, ösenförmige Aufnahme für ein flexibles Befestigungselement in einer variablen Anordnung nach Figur 5.

Figur 8 zeigt in Front- und Seitenansicht sowie in Draufsicht eine aus einem tiefgezogenen, randnahen Näpfchen geschaffene, ösenförmige Aufnahme für ein flexibles Befestigungselement in einer variablen Anordnung nach Figur 5.

Figur 9 zeigt in Front- und Seitenansicht sowie in Draufsicht eine aus überstehendem, randnahem Plattenmaterial stegförmig ausgeschnittene, hakenförmig gebogene Aufnahme für ein flexibles Befestigungselement in einer variablen Anordnung nach Figur 5.

Figur 10 zeigt in Front- und Seitenansicht sowie in Draufsicht eine aus überstehendem, randnahem Plattenmaterial flanschförmig ausgeschnittene, gebogene Aufnahme für ein flexibles Befestigungselement in einer variablen Anordnung nach Figur 5.

Figur 11 zeigt in Front- und Seitenansicht sowie in Draufsicht eine aus randnahem Plattenmaterial schräg eingeschnittene, abgewinkelte dreieckförmige Aufnahme für ein flexibles Befestigungselement in einer variablen Anordnung nach Figur 5.

Figur 12 zeigt die stegförmige Aufnahme für ein flexibles Befestigungselement der vergrößerten Schnittdarstellung B-B der Seitenansicht gemäß Figur 2.

Figur 13 zeigt in Frontansicht eine-dreiteilige, löffelförmige Implantatplatte mit quer zur Längsachse A-A verlaufenden Materialschwächungsbereichen oder selbsthemmenden Verhakungsscharnieren zwischen den Klingen-, Löffel- und Schaftabschnitten.

Figur 14 zeigt in Seitenansicht eine dreiteilige Implantatplatte mit Filmscharnieren zwischen der Klinge und dem kopfseitigen Abschnitt sowie zwischen dem kopfseitigen Abschnitt und dem schaftseitigen Abschnitt.

Figur 15 zeigt in Seitenansicht eine dreiteilige Implantatplatte mit stoßseitigen, scharnierartigen Verhakungen zwischen der Klinge und dem kopfseitigen Abschnitt sowie zwischen dem kopfseitigen Abschnitt und dem schaftseitigen Abschnitt.

Figur 16 zeigt den Verfahrensablauf und einen linearen, modularen Aufbau der Anlage zur Herstellung der erfindungsgemäßen Implantatplatten.

Gegebenenfalls nicht eingetragene Bezugszeichen ergeben sich aus den vorhergehenden oder nachfolgenden Figuren und den dazugehörigen Beschreibungen.

Die Erfindung umfaßt den konstruktiven Aufbau einer Implantatplatte zur Stabilisierung einer Fraktur eines Oberarmkopfes und/oder einer Fraktur des proximalen Oberarms, wobei die Implantatplatte neben den Bohrungen für die Oberarmkopfschrauben und Schaftschrauben auf der dem Knochen abgewandten Seite des Kopfabschnitts randnah angeordnete Aufnahmen für flexible Befestigungselemente wie Drahtcerclagen oder chirurgisches Nahtmaterial aufweist. Die Implantatplatte besitzt im wesentlichen eine durchgehend gleiche Materialstärke von ca. 0,5 bis 6,5 mm, vorzugsweise von 0,8 bis 3,5 mm. Der kopfseitige Abschnitt der Implantatplatte ist löffelförmig ausgeführt. Der schaftseitige Abschnitt der Implantatplatte ist gegenüber dem kopfseitigen Abschnitt flachstilförmig verschmälert ausgeführt Außerdem ist die löffelförmige Implantatplatte schwach konvex gebogen und im Querschnitt schwach bombiert bzw. hohlkehlförmig bzw. als flaches Rinnenprofil ausgeführt. Der kopfseitige Abschnitt besitzt mindestens eine Bohrung und/oder Langlochbohrung für Oberarmkopfschrauben. Der schaftseitige Abschnitt weist mindestens eine Bohrung und/oder Langlochbohrung für Schaftschrauben auf Auf dem löffelförmigen Abschnitt der Implantatplatte sind ausführungsgemäß randnah mindestens drei auf der dem Knochen abgewandten Seite abstehende, als Stege ausgebildete Aufnahmen für flexible Befestigungselemente vorgesehen. Die Aufnahmen besitzen jeweils eine Bohrung bzw. eine ösenförmige Aussparung zur Durchfiihrung eines flexiblen Befestigungselements. Diese ösenförmigen Aufnahmen erleichtern das schnelle Durchfädeln des flexiblen Befestigungselements wie Drahtcerclage oder chirurgisches Nahtmaterial.

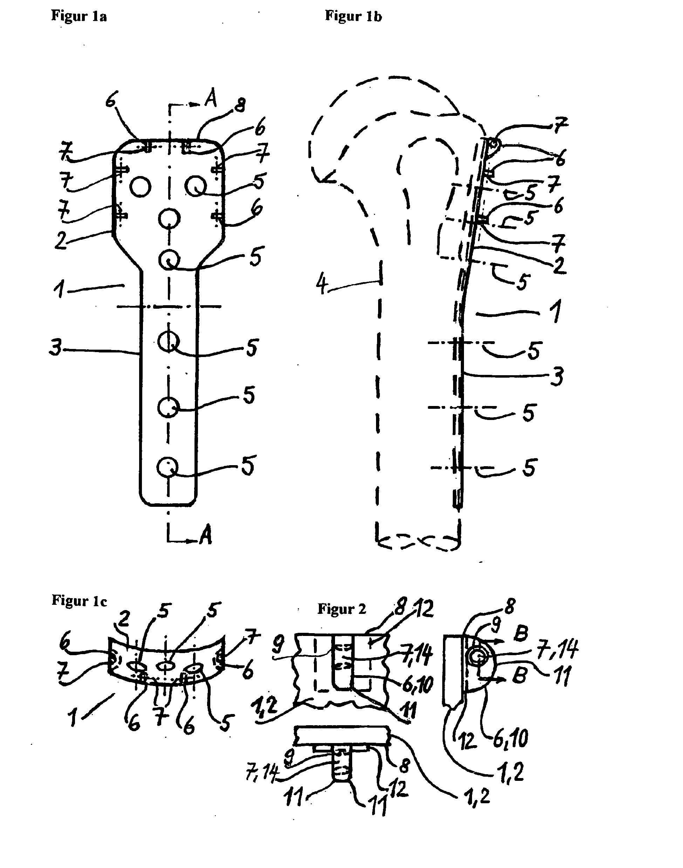

In den Figuren 1a, 1b und 1c ist eine erfindungsgemäße Implantatplatte 1 dargestellt, die einen kopfseitigen Abschnitt 2 und einen schaftseitigen Abschnitt 3 aufweist. Der kopfseitige Abschnitt 2 weist in bezug auf die Längsachse A-A der Implantatplatte 1 zwei davon gleichmäßig beabstandete Bohrungen 5 und zwei auf der Längsachse A-A liegende weitere Bohrungen 5 für Oberarmschrauben auf. Der schaftseitge Abschnitt 3 weist drei, auf der Längsachse A-A gleichmäßig beabstandet angeordnete Bohrungen 5 für Schaftschrauben auf. Der kopfseitige Abschnitt 2 besitzt auf der dem Knochen 4 abgewandten Oberfläche der Implantatplatte 1 sechs im wesentlichen gleichmäßig verteilte, stegförmige Aufnahmen 6 mit Aussparungen 7, die als Bohrungen 14 für nicht dargestellte flexible Befestigungselemente ausgeführt sind. Die Aussparungen 7 müssen im wesentlichen parallel zur Ober- und Unterseite der Implantatplatte 1 sowie parallel zur Außenkante bzw. -kontur 8 des Kopfabschnitts 2 verlaufen, wodurch das Einfädeln und Führen der flexiblen Befestigungselemente sowie das Umschlingen und Zusammenziehen der Fraktur mit dem flexiblen Befestigungselement entscheidend verbessert wird. Die Implantatplatte 1 ist nach der dargestellten Ausführungsform einteilig ausgeführt und leicht konvex gebogen, um eine gute Anlage an den Oberarmkopf und den proximalen Oberarm zu erreichen.

In Figur 2 ist in Front- und Seitenansicht sowie in Draufsicht in vergrößerter Darstellung die als Steg 10 ausgeführte Aufnahme 6 gemäß den Figuren 1a, 1b und 1c dargestellt. Jeder Steg 10 ist im wesentlichen senkrecht zur Außenkante 8 des kopfseitigen Abschnitts 2 der Implantatplatte 1 ausgerichtet und weist eine die Aussparung 7 repräsentierende Bohrung 14 auf Die Außenränder 9 der Bohrung 14 sind abgerundet, damit das nicht dargestellte flexible Befestigungselement leicht eingefädelt und beschädigungsfrei geführt werden kann. Die Kanten 11 des Stegs 10 sind zur Schonung des Gewebes ebenfalls gebrochen bzw. abgerundet ausgeführt. Der Steg 10 ist entsprechend der Seitenansicht halbkreisfömrig ausgeführt. Der Steg 10 kann halbovalförmig, quadratisch oder rechteckig jeweils mit gebrochenen oder gerundeten Ecken und Kanten 11 ausgerührt sein, um das Gewebe zu schonen. Der Steg 10 kann auf einer plattenfönnigen runden, quadratischen, rechteckförmigen, dreieckförmigen oder ovalen Basis 12 angeordnet sein. Die Basis 12 kann auf der dem Knochen 4 abgewandten Seite der Implantatplatte 1 randnah aufgeschweißt, preßgeschweißt, aufgelötet oder aufgeschraubt sein. Die Basis 12 kann auch entfallen, so daß der Steg 10 unmittelbar randnah auf der vorgesehenen Stelle der Implantatplatte 1 entsprechend befestigt sein kann.

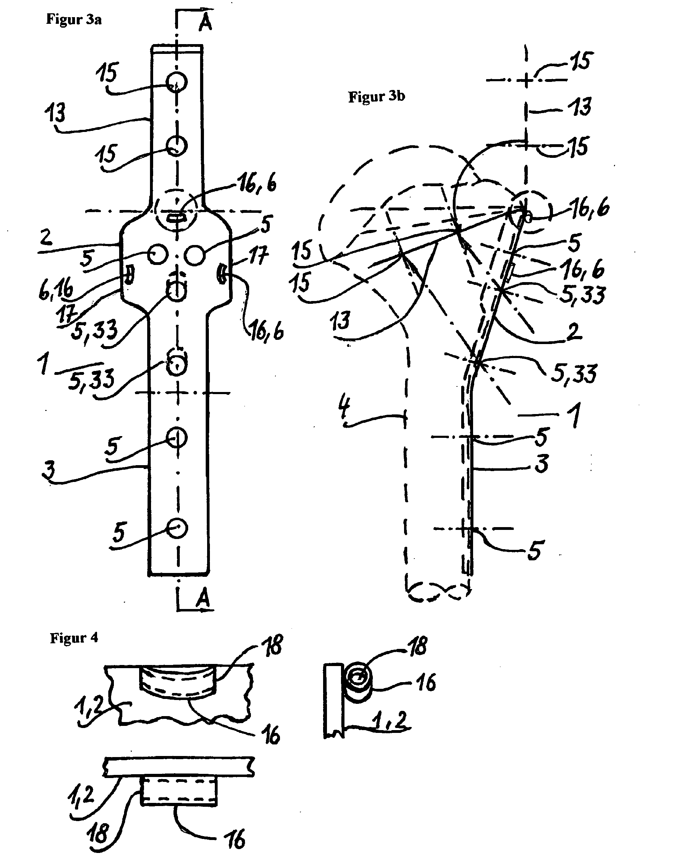

In Figuren 3a und 3b ist eine löffelförmige Implantatplatte 1 dargestellt, wobei von dem kopfseitigen Abschnitt 2 eine auf der Verlängerung der Längsachse A-A der Implantatplatte liegende Klinge 13 mit einer endseitigen scharfen Kante ausgeht. Auf dieser Längsachse A-A der Implantatplatte 1 sind im Abschnitt der Klinge 13 zwei beabstandete Bohrungen 15 mit einem eingeschnittenen Gewinde, das mindestens einen Gang aufweist, vorgesehen. Die Klinge 13 kann in bezug auf die vorliegende Fraktur des Oberarmkopfes entsprechend abgewinkelt und in den Oberarmkopf eingetrieben werden. Der kopfseitige Abschnitt 2 der Implantatplatte 1 weist zwei von der Längsachse A-A gleichmäßig beabstandete Bohrungen 5 und zwei auf der Längsachse A-A liegende Bohrungen 5 für Oberarmkopfschrauben auf Der schaftseitige Abschnitt 3 weist drei, auf der Längsachse A-A gleichmäßig beabstandete Bohrungen 5 für Schaftschrauben auf. Die beiden auf der Längsachse A-A beabstandet angeordneten Bohrungen 5 können auch als Langlöcher 33 für die Oberarmkopfschrauben ausgeführt sein. Nach der dargestellten Ausführungsform können zwei Oberarmkopfschrauben, insbesondere die beiden auf der Längsachse A-A liegenden Oberarmkopfschrauben, auf die beiden Bohrungen 15 der Klinge 13 ausgerichtet und in das mindestens eingängige Gewinde der Bohrungen 15 eingeschraubt werden. Diese Ausführungsform ermöglicht die Verriegelung der Implantatplatte gegen das Herauswandern aus dem Humeruskopf, so daß eine stabile Winkelverbindung zwischen dem Knochen 4 bzw. Humeruskopf, Klinge 13 und Implantatplatte 1 geschaffen wird. In Verbindung mit den durch die Aufnahmen 16 geführten flexiblen Befestigungselemente 29 und der drei randnah angeordneten rohrförmigen Aufnahmen 16 wird eine äußerst wirkungsvolle Fraktur-Stabilisierung bei wesentlich verkürzter Operationsdauer erreicht.

In Figur 4 ist in Front- und Seitenansicht sowie in Draufsicht in vergrößerter Darstellung die rohrförmige Aufnahme 16 nach den Figuren 3a und 3b dargestellt. Eine rohrförmige Aufnahme 16 ist am klingenseitigen Randbereich des kopfseitigen Abschnitts 2 der Implantatplatte 1 quer zur Längsachse A-A und parallel zur Oberseite der Implantatplatte 1 angeordnet. Außerdem ist je eine rohrförmige Aufnahme 16 an den gegenüberliegenden Längsseiten 17 des kopfseitigen Abschnitts 2 randnah sowie im wesentlichen parallel zur Längsseite 17 und parallel zur Oberseite der Implantatplatte 1 angeordnet. Die Ränder der Austrittsöffnungen 18 der rohrförmigen Aufnahmen 16 sind abgerundet. Die Austrittsöffnungen 18 der rohrförmigen Aufnahmen 16 weisen zur jeweiligen Außenkante bzw. -kontur 8 der Implantatplatte 1.

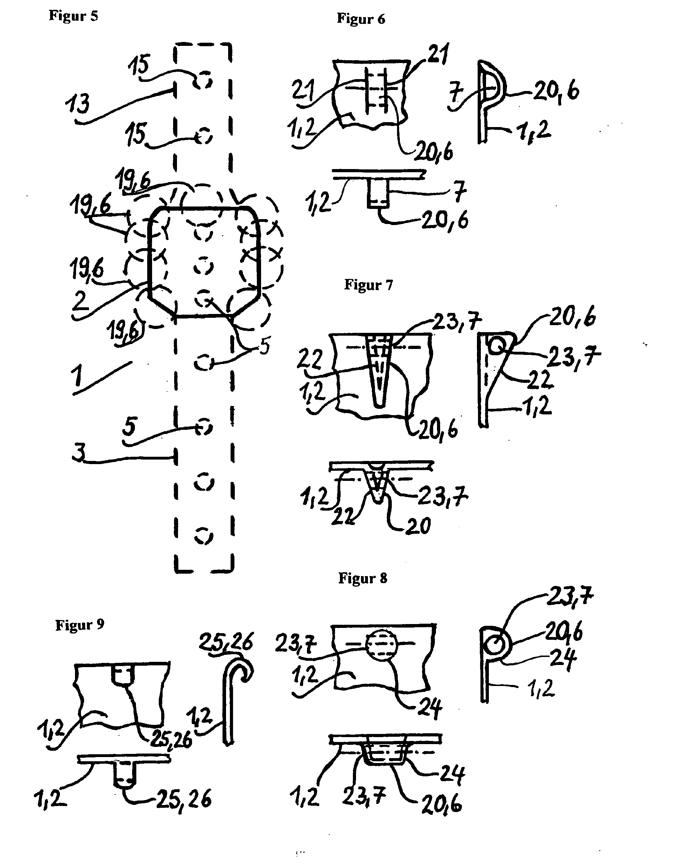

In Figur 5 ist der kopfseitige Abschnitt 2 der Implantatplatte 1 mit Klinge 13 und schaftseitigem Abschnitt 3, beide mit Unterbrechungslinien nur angedeutet, dargestellt. Im Bereich der Außenkante bzw. -kontur 8 des kopfseitigen Abschnitts 2 sind variable Platzhalterpositionen 19 für weitere Ausführungsformen von Aufnahmen 6 für flexible Befestigungselemente 29 vorgesehen, die mit kreisförmigen Unterbrechungslinien angedeutet sind.

In Figur 6 ist in Front- und Seitenansicht sowie in Draufsicht eine Aufnahme 20 mit ösenförmiger Aussparung 7 für flexible Befestigungselemente 29 entsprechend den Positionierungsmöglichkeiten 19 gemäß Figur 5 dargestellt. Diese Aufnahme 20 mit ösenförmiger Aussparung 7 ist aus einem randnahen Parallelschlitz 21 der Implantatplatte 1 durch Tiefziehen hergestellt. Sämtliche Kanten, die mit dem flexiblen Befestigungselement. 29 und mit dem Gewebe in Berührung kommen, sind abgerundet

In Figur 7 ist in Front- und Seitenansicht sowie in Draufsicht eine weitere Aufnahme 20 mit ösenformiger Aussparung 7 für flexible Befestigungselemente 29 entsprechend den Positionierungsmöglichkeiten 19 gemäß Figur 5 dargestellt. Diese ösenförmige Aussparung 7 der Aufnahme 20 ist durch Tiefziehen eines randnahen Bereichs der Implantatplatte 1 zu einer Falte 22 und durch Erstellen einer durchgehenden Bohrung 23 hergestellt. Sämtliche Kanten, die mit dem flexiblen Befestigungselement 29 und mit dem Gewebe in Berührung kommen, sind abgerundet.

In Figur 8 ist in Front- und Seitenansicht sowie in Draufsicht eine weitere Aufnahme 20 mit ösenförmiger Aussparung 7 für flexible Befestigungselemente 29 entsprechend den Positionierungsmöglichkeiten 19 gemäß Figur 5 dargestellt. Diese Aufnahme 20 mit der ösenförmigen Aussparung 7 ist durch Tiefziehen eines randnahen Bereichs der Implantatplatte 1 zu einem Näpfchen 24 und durch Erstellen einer durchgehenden Bohrung 23 hergestellt. Sämtliche Kanten, die mit dem flexiblen Befestigungselement 29 und mit dem Gewebe in Berührung kommen, sind abgerundet.

In Figur 9 ist in Front- und Seitenansicht sowie in Draufsicht eine rundhakenförmige Aufnahme 25 für flexible Befestigungselemente 29 entsprechend den Positionierungsmöglichkeiten gemäß Figur 5 dargestellt. Diese rundhakenförmige Aufnahme 25 ist durch Stanzen eines schmalen streifenförmigen Fortsatzes 26 aus einem überstehenden Randbereich der Implantatplatte 1 und anschließendes Rollen des freien Endes des Fortsatzes 26 zu einem Rundhaken 25 hergestellt. Sämtliche Kanten, die mit dem flexiblen Befestigungselement 29 und mit dem Gewebe in Berührung kommen können, sind abgerundet.

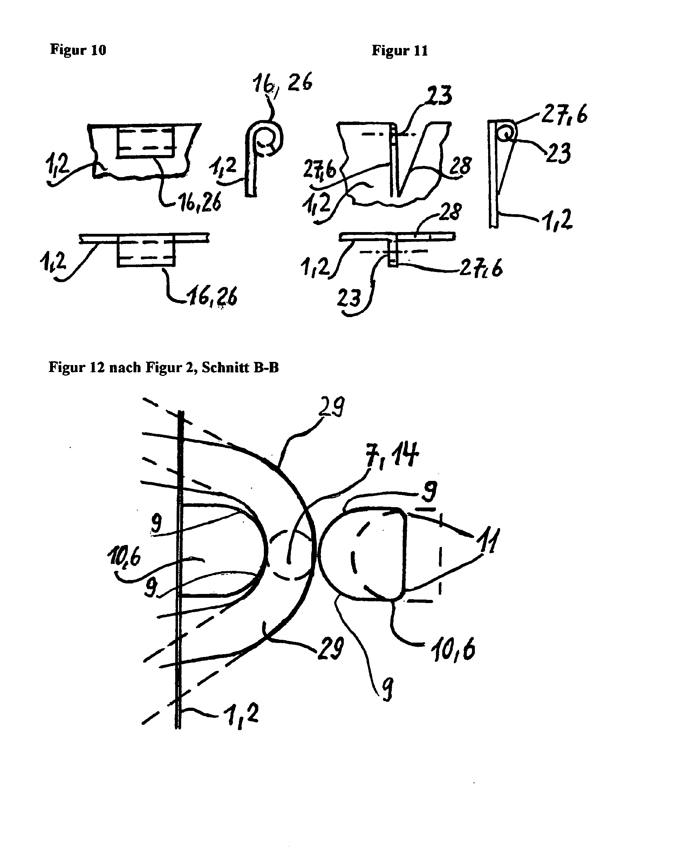

In Figur 10 ist in Front- und Seitenansicht sowie in Draufsicht eine andere rohrförmige Aufnahme 16 für flexible Befestigungselemente 29 entsprechend den Positionierungsmöglichkeiten gemäß Figur 5 dargestellt. Diese rohrförmige Aufnahme 16 ist durch Stanzen eines breiten rechteckförmigen Fortsatzes 26 aus einem überstehenden Randbereich der Implantatplatte 1 und anschließendes Rollen des freien Endes des Fortsatzes 26 zu einer rohrförmigen Aufnahme 16 hergestellt. Sämtliche Kanten, die mit dem flexiblen Befestigungselement 29 und mit dem Gewebe in Berührung kommen können, sind abgerundet.

In Figur 11 ist in Front- und Seitenansicht sowie in Draufsicht eine dreieckförmige, mit einer Bohrung 23 versehene Aufnahme 27 dargestellt, die durch einen Schrägschnitt 28 im randnahen Bandmaterial 42 und anschließendes, im wesentlichen vertikales Abwinkeln zur Oberseite der Implantatplatte 1 gebildet ist Sämtliche Kanten, die mit dem flexiblen Befestigungselement 29 und mit dem Gewebe in Berührung kommen, sind abgerundet.

Die Figur 12 zeigt den Steg 10 gemäß Schnitt B-B der Seitenansicht in Figur 2 in vergrößerter Darstellung. Danach ist die Aussparung 7 bzw. Bohrung 14 mit einem flexiblen Befestigungselement 29 belegt. Die Bohrung 14 kann auf den Durchmesser des flexiblen Befestigungselements 29 abgestimmt aber auch vergrößert sein, um das Einfädeln und ggf mehrfache Durchfädeln des flexiblen Befestigungselements 29 zu erleichtern.

Sämtliche Kanten 8, 9 der Implantatplatte 1 auf der Basis der erfindungsgemäßen Ausführungsformen, die mit dem flexiblen Befestigungselement 29 und mit dem Gewebe in Berührung kommen, sind gebrochen, abgerundet und/oder geglättet.

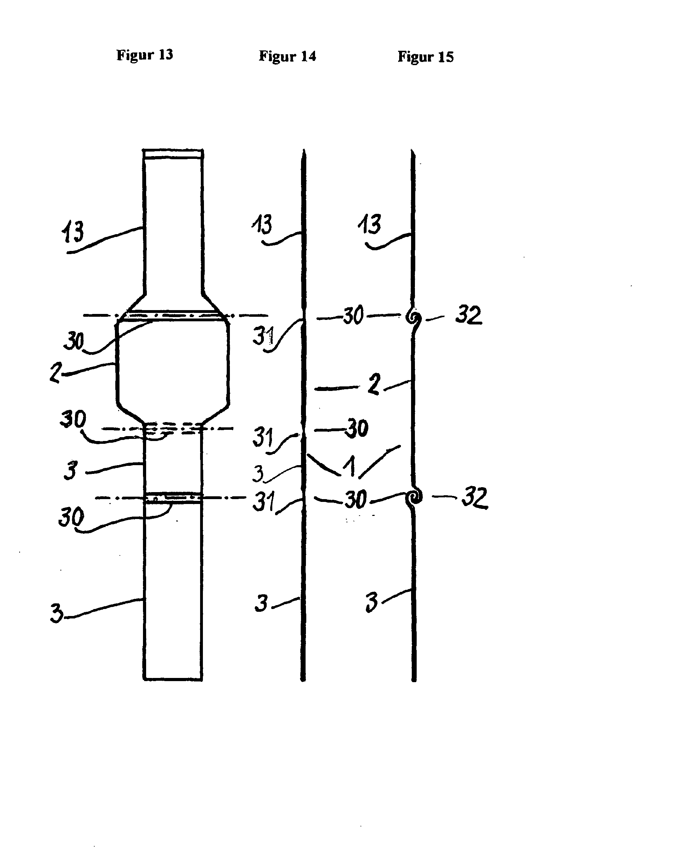

In Figur 13 ist in Frontansicht eine dreiteilige, löffelförmige Implantatplatte 1 mit mindestens zwei, quer zur Längsachse A-A verlaufenden Materialschwächungsbereichen 30 zwischen den Kopf-, Schaft- und Klingenabschnitten 2, 3, 13 dargestellt. Die beiden Materialschwächungsbereiche 30 werden beim chirurgischen Einsatz der Implantatplatte 1 als Filmscharniere 31 oder Trennungsstellen zur Bildung von Verhakungsscharnieren 32 genutzt.

In Figur 14 sind in Seitenansicht drei Materialschwächungsbereiche 30 der Implantatplatte 1 dargestellt, die im späteren chirurgischen Einsatz als Filmscharniere 31 zur verbesserten Anpassung an die Form des Oberarmkopfes und proximalen Oberarms genutzt werden können; denn auf diese Weise kann die gewünschte Abwinkelung des kopf- und klingenseitigen Abschnitts erreicht werden.

In Figur 15 sind in Seitenansicht zwei Materialschwächungsbereiche 30 der Implantatplatte 1 dargestellt, die im späteren chirurgischen Einsatz als selbsthemmende Verhakungsscharniere 32 zur verbesserten Anpassung an die Form des Oberarmkopfes und proximalen Oberarms genutzt werden können; denn auch nach dieser Ausführungsform wird die gewünschte Abwinkelung des kopf- und klingenseitigen Abschnitts erreicht.

Die erfindungsgemäße Implantatplatte 1 kann durch Entfall der Klinge 13 nur zweiteilig ausgeführt sein, wobei der Materialschwächungsbereich 30 zwischen Kopfabschnitt 2 und Schaftabschnitt 3 als Filmscharnier 31 oder selbsthemmendes Verhakungsscharnier 32 für die Abwinkelung und Anpassung an den Knochen 4 genutzt wird. Bei der dreiteiligen Ausführungsform der Implantatplatte 1 können die Materialschwächungsbereiche 30 in Kombination als Filmscharnier 31 und selbsthemmendes Verhakungsscharnier 32 verwendet werden.

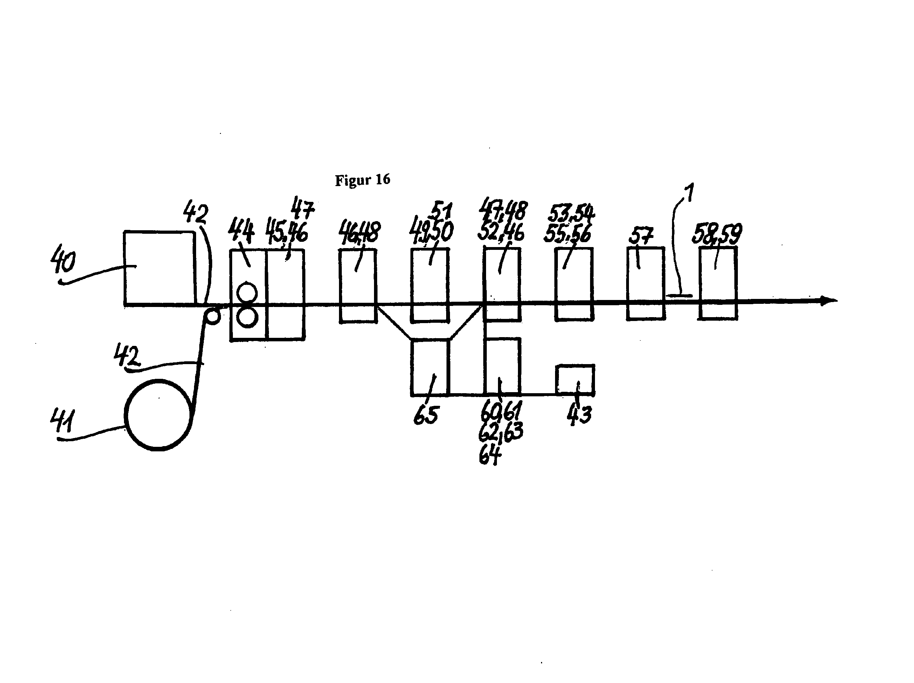

In Figur 16 ist das Verfahren im Rahmen einer Anlage zur Herstellung der Implantatplatten 1 dargestellt. Danach umfaßt das Verfahren eine halb- oder vollautomatische Herstellung von Implantatplatten 1, welche zur Stabilisierung einer Fraktur eines Oberarmkopfes und/oder einer Fraktur des proximalen Oberarms verwendet werden. Hierzu wird ein humankörperverträgliches, abgelängtes und in einem Magazin 40 gestapeltes oder als Coil 41 vorliegendes metallisches Bandmaterial 42, das beispielsweise aus Implantatstahl, Titan oder Titanlegierungen besteht, als Ausgangsmaterial für die Implantatplatte 1 verwendet. Das Bandmaterial 42 weist eine weitgehende gleichbleibende Stärke bzw. Dicke von 0,5 bis 6,5 mm auf. Die Bandbreite richtet sich nach der jeweiligen maximalen Breite des kopfseitigen Abschnitts 2 der löffelförmigen Implantatplatte
1. Die Breite kann auch zwecks Bildung von Aufnahmen 6 aus dem Bandmaterial 42 breiter ausgeführt sein. Die zu verwendenden Bandbreiten bestimmen sich im übrigen nach der Verwendung der Implantatplatten 1 für Erwachsene, Jugendliche und Kinder.

Das Bandmaterial 42, das aus dem Magazin 40 oder von der Abcoilvorrichtung 43 bereitgestellt wird, kann nach einer Ausführungsform rechnergesteuert und getaktet ein Walzwerk 44 durchlaufen, das mit einem Bohrwerk 45 und/oder einer Laservorrichtung 46 kombiniert sein kann. Die Oberfläche einer Walze des Walzwerks 44 kann profiliert sein und auf der später dem Knochen 4 zugewandten Seite des Bandmaterials 42 der herzustellenden Implantatplatte 1 beispielsweise ein Noppen-Profil oder unterbrochene Rillen-, Rauten- udgl Profile erzeugen. Das Bohrwerk 45 und/oder die Laservorrichtung 46 führen programmgesteuert die notwendigen Bohrungen 14, 23 aus, welche die Aussparungen 7 in den Aufnahmen 6 für die flexiblen Befestigungselemente 29 bilden. Das Bandmaterial 42 wird daraufhin in einer Stanzvorrichtung 47 bearbeitet, die mit der Laservorrichtung 46 und/oder Schneidvorrichtung 48 kombiniert sein kann. In diesen Vorrichtungen werden beispielsweise programmgesteuert Parallelschlitze 21, randnahe Fortsätze bzw. Streifen 26 udgl. hergestellt. Anschließend wird das Bandmaterial 42 rechnergesteuert und getaktet in einer Tiefziehvorrichtung 49 bearbeitet, die mit einer Biegevorrichtung 50 und/oder Rollvorrichtung 51 kombiniert sein kann, um randnahe Parallelschlitze 21 oder Näpfchen 24 zu einer Öse oder randnahe Plattenbereiche zu Falten 22 zu verformen oder randnahe Schrägschnitte 28 abzuwinkeln oder überstehende Materialstreifen 26 einzurollen. Danach wird in einer Trennvorrichtung 52, wobei auf die Laservorrichtung 46, die Stanzvorrichtung 47 und/oder die Schneidvorrichtung 48 zurückgegriffen werden kann, programmgesteuert die löffelförmige Endkonfiguration und die Vereinzelung des Bandmaterials 42 zu Implantatplattenrohlingen 1 durchgeführt. Zur Erzielung einer verbesserten Anlage der Implantatplatte 1 an den Knochen 4 kann das Bandmaterial 42 vor oder nach der Vereinzelung zu Implantatplattenrohlingen 1 in einer Kaltreckvorrichtung 65 zu einem flachen Rinnenprofil kaltumgeformt werden. Hierfür kann auch das Walzwerk 44 durch entsprechende Umrüstung und reversieren der Implantatplattenrohlinge 1 genutzt werden. Abschließend werden die Implantatplatten 1 einer Abgratvorrichtung 53 in Kombination mit einer Abstrahlvorrichtung 54, Rundungsvorrichtung 55 und Säuberungsvorrichtung 56 zugeführt. Danach können die Implantatplatten 1 in einer Beschichtungsvorrichtung 57 oberflächenveredelt werden. Die Veredelung der Implantatplatte 1 kann mit galvanischen, elektrochemischen oder vakuumtechnischen Beschichtungstechniken erfolgen. Die vakuumtechnische Beschichtung umfaßt vor allem die CVD-Technik (chemical vapour deposition). Abschließend werden die Implantatplatten 1 in einem Sterilisator 58 in Verbindung mit Verpackungsvorrichtungen 59 versandfertig gemacht und dem Vertrieb zugeleitet.

Die zur Erfindung gehörende Anlage ist modular aufgebaut und umfaßt im wesentlichen folgende Vorrichtungen, insbesondere programmgesteuerte, halb- oder vollautomatisch und getaktet arbeitende Fertigungsmaschinen oder -automaten, mit denen die Vorbereitung des metallischen Bandmaterials 42 und die Bildung der Aufnahmen 6 für flexible Befestigungselemente 29 sowie der Endkonfiguration der Implantatplatten 1 ggf mit Klinge 13 durchgeführt wird:
a) wenigstens ein Stapelmagazin 40 und/oder wenigstens eine Abcoilvorrichtung 41 ggf mit Umlenkrolle für die Bereitstellung und Zuführung des metallischen Bandmaterials 42;
b) ggf. ein Walzwerk 44 zur wenigstens einseitigen Oberflächenprofilierung des Bandmaterials 42, z.B. zur Erzeugung von Noppen, unterbrochenen Rollen, Rauten udgl. Profile auf der dem Knochen 4 zugewandten Seite;
c) ggf. eine Bohr-, Laser- und/oder Stanzvorrichtung 45, 46, 47, insbesondere ein entsprechender Automat für die Herstellung von Aussparungen 7, Lochsetzungen bzw. Bohrungen 23 udgl.;
d) ggf. eine Laser-, Stanz- und/oder Schneidvorrichtung 46, 47, 48, insbesondere ein entsprechender Automat zur Vorbereitung des Flachbandes für die Bildung erhabener Aufnahmen 6 auf der dem Knochen 4 abgewandten Seite der Implantatplatte 1 sowie ggf einer vom löffelförmigen Abschnitt 2 des Implantatplattenrohlings 1 ausgehenden Klinge 13;
e) ggf Tiefzieh-, Biege- und Rollvorrichtungen 49, 50, 51, insbesondere ein entsprechender Automat für die Formgebung der erhabenen Aufnahmen 6 auf der dem Knochen 4 abgewandten Seite des Implantatplattenrohlings 1 sowie ggf. der U-, T- oder I-förmigen Profilierung der Klinge 13 durch Kaltumformung;
f) ggf. weitere Laser-, Stanz und/oder Schneidvorrichtungen 46, 47, 48, insbesondere ein entsprechender Automat für die Herstellung der Außenkontur 8 und Vereinzelung der Implantatplattenrohlinge 1;
g) ggf. mindestens ein Kleinteilemagazin 43 für extern hergestellte Ösen, Rundhaken oder Rohrabschnitte;
h) ggf Schweiß-, Preßschweiß-, Löt-, Schraub- und/oder Nietvorrichtungen 60, 61, 62, 63, 64, insbesondere ein entsprechender Automat für das Befestigen extern hergestellter Aufnahmen 6 für flexible Befestigungselemente 29 auf der dem Knochen 4 abgewandten Seite des Implantatplattenrohlings 1,
i) ggf eine Kaltreckvorrichtung 65, insbesondere ein entsprechender Automat zur Erzeugung von quer zur Längsachse verlaufenden Materialschwächungsbereichen 30 und Anschärfung des freien Endes der Klinge 13 sowie Überführung des Bandmaterials 42 in ein flaches, die Klingenbreite im wesentlichen nicht mehr erfassendes, rinnenförmiges Profil,
j) Abgrat-, Rundungs-, Abstrahl- und/oder Säuberungsvorrichtungen 53, 54, 55, 56, insbesondere ein entsprechender Automat zur Beseitigung scharfer Kantenbereiche 9 auf den Implantatplatten 1,
k) ggf eine Beschichtungsvorrichtung 57 zur Oberflächenveredelung, insbesondere galvanische, elektrochemische und vakuumtechnische Beschichtungsvorrichtungen, sowie
l) ein Sterilisator 58 und/oder Sterilverpackungsvorrichtung 59, insbesondere einen entsprechenden Automaten.

Nach der dargestellten anlagetechnischen Ausführungsform sind die einzelnen Vorrichtungen in linearer Ausrichtung angeordnet. Die Vorrichtungen der Anlage können auch linear gegenüberliegend oder mäanderförmig versetzt angeordnet sein. Schließlich kann die Anlage als Karussell, Drehkreuz oder Doppelstern aufgebaut sein, wobei die einzelnen Vorrichtungen funktionsgemäß zusammenarbeiten. Automaten mit Mehrfachfunktionen können verfahrens- und anlagetechnisch wiederholt, ggf. nach erforderlicher Umrüstung, eingesetzt werden, so daß der anlage- und vorrichtungstechnische Aufwand zur Bewältigung der einzelnen Verfahrensschritte durch Reversieren des Bandmaterials 42 oder der Implantatplattenrohlinge 1 reduziert werden kann.

Nach einer weiteren Ausführungsform können die einzelnen Materialschwächungsbereiche 30 der Implantatplatten 1 als Filmscharniere 31 genutzt werden. Die verfahrens- und anlagetechnische Herstellung erfolgt vorzugsweise in einer separaten Kaltreckvorrichtung 65. Auch das Walzwerk 44 kann durch entsprechende Umrüstung für die Kaltreckung des Bandmaterials 42 genutzt werden, um programmgesteuert Materialschwächungsbereiche 30 in dem Bandmaterial 42 herzustellen.

Die Herstellung der zwei- oder dreiteiligen Implantatplatten 1 erfolgt in der Weise, daß das Bandmaterial 42 entsprechend der klingen-, kopf- und/oder schaftseitigen Abschnitte 2, 3, 13 durch Stanzen oder Lasern voneinander getrennt werden. Die stoßseitigen Kanten der vereinzelten Abschnitte 2, 3, 13 werden als selbsthemmende Verhakungsscharniere 32 ausgeführt und durch Ineinanderstecken begrenzt beweglich, miteinander verbunden.

Auf diese Weise wird eine kostengünstige Herstellung der Implantatplatten 1 fertigungstechnisch ermöglicht.

Die Erfindung ermöglicht auch die Verwendung der Implantatplatten 1 für die Stabilisierung von Frakturen des Oberarmkopfes und/oder des proximalen Oberarms, wobei das Fixieren der Fraktur mit der Implantatplatte 1, den Kopf- und Schaftschrauben sowie mit den um die Frakturteile, insbesondere die Tubercula geschlungenen flexiblen Befestigungselemente 29 in Verbindung mit den auf der Oberseite der Implantatplatte 1 randnah angeordneten Aufnahmen 6 erfolgt. Die Aussparungen 7 der Aufnahmen 6 verlaufen nach der Erfindung im wesentlichen parallel zur Außenkontur 8 des Kopfabschnittes 2.

Mit der Erfindung werden erhebliche chirurgische Vorteile erzielt. Sie ermöglicht mit den Aufnahmen 6 für die flexiblen Befestigungselemente 29, die Drahtcerclagen oder chirurgisches Nahtmaterial umfassen, ein wesentlich einfacheres und schnelleres Ein- und Durchfädeln sowie Zusammenziehen. Mit dieser konstruktiven Lösung wird eine wesentlich bessere Fraktur-Fixierung und Schonung des Knochenmatrials erreicht. Außerdem wird eine wesentliche Verkürzung der Operationsdauer erzielt. Der Patient wird dadurch physiologisch entlastet, weil der Narkoseeinsatz und Blutverluste verringert werden.

## Patentansprüche

1. Implantatplatte mit Bohrungen für Knochenschrauben zur Stabilisierung einer Fraktur eines Oberarmkopfes und/oder einer Fraktur des proximalen Oberarms, umfassend
eine löffelförmige Implantatplatte (1), einen kopfseitigen Abschnitt (2) und einen schaftseitigen Abschnitt (3) jeweils mit mindestens einer Bohrung (5) und/oder
einer Langlochbohrung (5),
**dadurch gekennzeichnet, dass**
mindestens eine erhabene Aufnahme (6) für ein flexibles Befestigungselement (29) auf der dem Knochen (4) abgewandten Seite des kopfseitigen Abschnitts (2) der Implantatplatte (1) an deren Außenkante bzw. -kontur (8) angeordnet ist,
jede Aufnahme (6) eine zu der Außenkante bzw. -kontur (8) und der dem Knochen (4) abgewandten Seite des kopfseitigen Abschnitts (2) der Implantatplatte (1) im wesentlichen parallel verlaufende Aussparung (7) für das Ein- Durchfädeln sowie Zusammenziehen des flexiblen Befestigungselements (29) besitzt.

2. Implantatplatte nach Anspruch 1,
**dadurch gekennzeichnet, daß**
der Außenrand (9) der Aussparung (7) in der Aufnahme (6) für das flexible Befestigungselement (29) ein- und austrittsseitig gebrochen, gerundet und/oder geglättet ist.

3. Implantatplatte nach den Ansprüchen 1 und 2,
**dadurch gekennzeichnet, daß**
die Implantatplatte (1) mit dem kopfseitigen Abschnitt (2) und dem schaftseitigen Abschnitt (3) im wesentlichen die gleiche Materialstärke aufweist,
der kopfseitige Abschnitt (2) der Implantatplatte (1) löffelförmig verbreitert und
der schaftseitige Abschnitt (3) demgegenüber schmäler ausgeführt ist, sowie
jede Aufnahme (6) für das flexible Befestigungselement (29) an der Außenkante bzw. -kontur (8) des kopfseitigen Abschnitts (2) beabstandet angeordnet ist.

4. Implantatplatte nach den Ansprüchen 1 bis 3,
**dadurch gekennzeichnet, daß**
die Aussparung (7) der Aufnahme (6) als Bohrungen oder ösen-, rohr- oder rundhakenförmig ausgeführt ist.

5. Implantatplatte nach den Ansprüchen 1 bis 4,
**dadurch gekennzeichnet, daß**
die Aufnahmen (6) aus Bandmaterial (42) durch Lasern, Stanzen, Schneiden, Tiefziehen, Biegen und Rollen hergestellt sind, und
die Aussparungen (7) durch Bohren, Stanzen, Lasern, Tiefziehen, Biegen und Rollen hergestellt sind.

6. Implantatplatte nach den Ansprüchen 1 bis 5,
**dadurch gekennzeichnet, daß**
die Aufnahmen (6) aus extern vorgefertigten Stegen (10) mit Bohrungen (14), rohrförmigen Aufnahmen (16) oder Rundhaken (25) mit oder ohne Basis (12) bestehen, und
die Aufnahmen (6) auf den vorbestimmten, randnahen Platzhalterpositionen (19) des Bandmaterials (42) aufgeschweißt, preßgeschweißt, gelötet, geschraubt oder genietet sind.

7. Implantatplatte nach den Ansprüchen 1 bis 6,
**dadurch gekennzeichnet, daß**
sämtliche Kanten und Ränder (8, 9, 11), die mit dem flexiblen Befestigungselement (29) und dem Humangewebe in Berührung kommen, gebrochen, abgerundet und/oder geglättet sind.

8. Implantatplatte nach den Ansprüchen 1 bis 7,
**dadurch gekennzeichnet, daß**
der kopfseitige Abschnitt (2) der Implantatplatte (1) eine auf der verlängerten Längsachse A-A liegende Klinge (13) mit endseitig scharfer Kante besitzt.

9. Implantatplatte nach Anspruch 8,
**dadurch gekennzeichnet, daß**
die Klinge (13) mindestens eine Bohrung (15) mit jeweils mindestens einem Gewindegang aufweist, in welche die von dem kopfseitigen Abschnitt (2) bzw. Kopfabschnitt (2) der Implantatplatte (1) ausgehenden Oberarmkopfschrauben einschraubbar sind.

10. Verfahren zur Herstellung von Implantatplatten für den Humerus,
**dadurch gekennzeichnet, daß**
eine löffelförmige Implantatplatte, die mit Bohrungen und/oder Langlöchern für Knochenschrauben ausgestattet ist, mit mindestens einer Aufnahme für ein flexibles Befestigungselement auf der dem Knochen abgewandten Seite der Implantatplatte versehen wird, und
die Aufnahme mit einer Aussparung, die im wesentlichen parallel zur Ober- und Unterseite sowie parallel zur Außenkante bzw. -kontur der Implantatplatte verläuft, ausgestattet wird.

11. Verfahren nach Anspruch 10,
**dadurch gekennzeichnet, daß**
ein humankörperverträgliches, metallisches Bandmaterial wie Implantatstahl, Titan oder Titanlegierungen abgelängt oder als Coil bereitgestellt wird,
das Bandmaterial in einer Bohr-, Laser- und/oder Stanzvorrichtung programmgesteuert bearbeitet wird, um die notwendigen Aussparungen mit relativ großem Durchmesser für die Knochenschrauben zu erstellen,
das Bandmaterial gleichzeitig oder anschließend in einer Bohr-, Laser- und/oder Stanzvorrichtung programmgesteuert bearbeitet wird, um die notwendigen Aussparungen mit relativ kleinem Durchmesser für flexible Befestigungselemente zu erstellen,
gleichzeitig oder anschließend die für die Bildung der Aufnahmen und ggf. einer Klinge erforderlichen randnahen Formschnitte wie Parallelschnitte, Schrägschnitte, Teilausschnitte udgl. in einer Stanz-, Laser-, Schneid- udgl. Vorrichtung erstellt werden,
die für die Bildung der randnahen Aufnahmen vorbereiteten Bereiche des Materialbandes in Richtung der dem Knochen abgewandten Seite des Implantats randnah tiefgezogen, gebogen, gerollt, abgewinkelt udgl. werden,
die durch Bohren, Stanzen, Lasern, Tiefziehen, Biegen, Rollen, Abwinkeln udgl. gebildeten Aussparungen der Aufnahmen im wesentlichen parallel zur Ober- und Unterseite sowie parallel zur Außenkante bzw. -kontur der Implantatplatte verlaufend angeordnet werden, und
sämtliche Kanten und Ränder der Implantatplatte gebrochen und/oder gerundet sowie geglättet sind.

12. Verfahren nach den Ansprüchen 10 und 11,
**dadurch gekennzeichnet, daß**
eine löffelförmige Implantatplatte, die mit Bohrungen für Knochenschrauben ausgestattet ist, mit mindestens einer extern gebildeten Aufnahme mit integrierter Aussparung für ein flexibles Befestigungselement auf der dem Knochen abgewandten Seite der Implantatplatte versehen wird, und
die Aussparung in der Aufnahme im wesentlichen parallel zur Ober- und Unterseite sowie parallel zur Außenkante bzw. -kontur der Implantatplatte angeordnet wird.

13. Anlage zur Herstellung von Implantatplatten nach Anspruch 9 oder zur Ausführung
eines Verfahrens nach Anspruch 11 oder 12,
**dadurch gekennzeichnet, daß**
a) wenigstens ein Stapelmagazin (40) oder wenigstens eine Abcoilvorrichtung (43) für die Bereitstellung und Zuführung des metallischen Bandmaterials (42),
b) ein Walzwerk (44) zur wenigstens einseitigen Oberflächenprofilierung des Bandmaterials (42), z.B. zur Erzeugung von Noppen, unterbrochenen Rollen, Rauten udgl. Profile auf der dem Knochen (4) zugewandten Seite,
c) eine Bohr-, Laser- und/oder Stanzvorrichtung (45, 46, 47), insbesondere ein entsprechender Automat für die Herstellung von Aussparungen (7), Lochsetzungen bzw. Bohrungen (14, 23) udgl.,
d) eine Laser-, Stanz- und/oder Schneidvorrichtung (46, 47, 48), insbesondere ein entsprechender Automat zur Vorbereitung des Bandmaterials (42) für die Bildung erhabener Aufnahmen (6) auf der dem Knochen (4) abgewandten Seite der Implantatplatte (1) sowie ggf. einer vom löffelförmigen Abschnitt (2) des Implantatplattenrohlings (1) ausgehenden Klinge (13),
e) Tiefzieh-, Biege- und Rollvorrichtungen (49, 50, 51), insbesondere ein entsprechender Automat für die Formgebung der erhabenen Aufnahmen (6) auf der dem Knochen (4) abgewandten Seite des Implantatplattenrohlings (1) sowie ggf. der U-, T- oder I-förmigen Profilierung der Klinge (13) durch Kaltumformung,
f) weitere Laser-, Stanz- und/oder Schneidvorrichtungen (46, 47, 48), insbesondere ein entsprechender Automat für die Herstellung der Außenkontur (8) und Vereinzelung der Implantatplattenrohlinge (1),
g) mindestens ein Kleinteilemagazin (43) für extern hergestellte Aufnahmen (6) z.B. Ösen, Haken oder Rohrabschnitte,
h) Schweiß-, Preßschweiß-, Löt-, Schraub- und/oder Nietvorrichtungen (60, 61, 62, 63, 64), insbesondere ein entsprechender Automat für das Befestigen extern hergestellter Aufnahmen (6) für flexible Befestigungselemente (29) auf der dem Knochen (4) abgewandten Seite des Implantatplattenrohlings (1),
i) eine Kaltreckvorrichtung (65), insbesondere ein entsprechender Automat zur Erzeugung von quer zur Längsachse verlaufenden Materialschwächungsbereichen (30) und Anschärfung des freien Endes der Klinge (13) sowie Überführung des Bandmaterials (42) in ein flaches, die Breite der Klinge (13) im wesentlichen nicht mehr erfassendes, rinnenförmiges Profil,
j) Abgrat-, Rundungs-, Abstrahl- und/oder Säuberungsvorrichtungen (53, 54, 55, 56), insbesondere ein entsprechender Automat zur Beseitigung scharfer Kantenbereiche (8, 9, 11) auf den Implantatplatten (1),
k) eine Beschichtungsvorrichtung (57) zur Oberflächenveredelung, insbesondere galvanische, elektrochemische und vakuumtechnische Beschichtungsvorrichtungen, sowie
l) ein Sterilisator (58) und/oder Sterilverpackungsvorrichtung (59), insbesondere ein entsprechender Automat,
eingesetzt werden,
wobei wahlweise die Merkmale d) und e) oder g) und h) anwesend sein müssen.

## Claims

1. An implant plate having holes for bone screws for stabilizing a fracture of an upper arm bone head and/or a fracture of the proximal upper arm, comprising a spoon-shaped implant plate (1), a head-side section (2), and a shaft-side section (3), each having at least one hole (5) and/or one oblong hole (5),
**characterized in that** at least one raised receiving member (6) for a flexible fastener (29) is situated on the side of the head-side section (2) of the implant plate (1) facing away from the bone (4), on its external edge and/or contour (8), each receiving member (6) has an opening (7), extending essentially in parallel to the outer edge and/or contour (8) and the side of the head-side section (2) of the implant plate (1) facing away from the bone (4), for threading through and tightening the flexible fastener (29).

2. The implant plate according to Claim 1,
**characterized in that** the external edge (9) of the opening (7) in the receiving member (6) for the flexible fastener (29) is blunted, rounded, and/or smoothed at the entry and exit.

3. The implant plate according to Claims 1 and 2,
**characterized in that** the implant plate (1) has essentially the same material thickness in the head-side section (2) and the shaft-side section (3),
the head-side section (2) of the implant plate (1) expands in a spoon shape and the shaft-side section (3) is implemented as narrower in relation thereto, and the receiving members (6) for the flexible fastener (29) are spaced along the outer edge and/or contour (8) of the head-side section (2).

4. The implant plate according to Claims 1 through 3,
**characterized in that** the opening (7) of the receiving member (6) is implemented as a hole or in the form of an eye, tube, or rounded hook.

5. The implant plate according to Claims 1 through 4,
**characterized in that** the receptacles (6) are produced from strip material (42) by at least one of laser treatment, punching, cutting, deep-drawing, bending, and edge rolling, and
the openings (7) are produced by drilling, punching, laser treatment, deep-drawing, bending, and edge rolling.

6. The implant plate according to Claims 1 through 5,
**characterized in that** the receiving members (6) comprise externally prefinished webs (10) having holes (14), tubular receptacles (16), or rounded hooks (25) with or without a base (12),
and the receiving members (6) are welded, pressure-welded, soldered, screwed, or riveted onto predetermined locating positions (19) of the strip material (42) in proximity to the boundary.

7. The implant plate according to Claims 1 through 6,
**characterized in that** all edges and boundaries (8, 9, 11) which come into contact with the flexible fastener (29) and the human tissue are blunted, rounded, and/or smoothed.

8. The implant plate according to Claims 1 through 7,
**characterized in that** the head-side section (2) of the implant plate (1) has a blade (13) lying on the lengthened longitudinal axis A-A having a sharp edge at the end.

9. The implant plate according to Claim 8,
**characterized in that** the blade (13) has at least one hole (15) having at least one thread, into which the upper arm head screws originating from the head-side section (2) and/or head section (2) of the implant plate (1) may be screwed.

10. A method for producing implant plates for the humerus,
**characterized in that** a spoon-shaped implant plate, which is equipped with holes and/or oblong holes for bone screws, is provided with at least one receiving member for a flexible fastener on the side of the implant plate facing away from the bone, and
the receiving member is equipped with an opening which extends essentially in parallel to the upper and lower sides and in parallel to the external edge and/or contour of the implant plate.

11. The method according to Claim 10,
**characterized in that** a metallic strip material such as implant steel, titanium, or titanium alloys, which is compatible with the human body, is cut to length or provided as a coil,
the strip material is processed program-controlled in a drilling, laser treatment, and/or punching device to prepare the required openings having relatively large diameters for the bone screws,
the strip material is simultaneously or subsequently processed in a drilling, laser treatment, and/or punching device which is program-controlled to produce the necessary apertures of relatively small diameters for flexible fasteners, simultaneously or subsequently, the shaped cuts in proximity to the boundary, such as parallel cuts, diagonal cuts, partial cutouts, and the like, which are required for forming the receptacles and possibly a blade, are prepared in a punching, laser treatment, or cutting device or the like,
the areas of the material strip prepared to form the receiving members in proximity to the boundary are deep-drawn, bent, rolled, angled, or the like in proximity to the boundary in the direction of the side of the implant facing away from the bone,
the openings of the receiving members formed by drilling, punching, laser treatment, deep-drawing, bending, rolling, angling, or the like extend essentially in parallel to the upper and lower sides and in parallel to the outer edge and/or contour of the implant plate, and
all edges and boundaries of the implant plate are blunted and/or rounded as well as smoothed.

12. The method according to Claims 10 and 11,
**characterized in that** a spoon-shaped implant plate which is equipped with holes for bone screws is provided with at least one externally formed receiving member having an integrated opening for a flexible fastener on the side of the implant plate facing away from the bone, and
the opening in the receptacle extends essentially in parallel to the upper and lower sides and in parallel to the outer edge and/or contour of the implant plate.

13. A facility for producing implant plates according to Claim 9 or for executing a method according to Claim 11 or 12,
**characterized in that**
a) at least one stack magazine (40) or at least one uncoiling device (43) for providing and feeding the metallic strip material (42),
b) a rolling mill (44) for surface profiling of the strip material (42) on at least one side, e.g., for generating nubs, interrupted rolls, rhomboids, or similar profiles on the side facing towards the bone (4),
c) a drilling, laser treatment, and/or punching device (45, 46, 47), in particular a corresponding automatic machine for producing openings (7), perforations and/or holes (14, 23), or the like,
d) a laser treatment, punching, and/or cutting device (46, 47, 48), in particular a corresponding automatic machine for preparing the strip material (42) for the formation of raised receiving members (6) on the side of the implant plate (1) facing away from the bone (4) and possibly a blade (13) originating from the spoon-shaped section (2) of the implant plate blank (1),
e) deep-drawing, bending, and rolling devices (49, 50, 51), in particular a corresponding automatic machine for the shaping of the raised receiving members (6) on the side of the implant plate blank (1) facing away from the bone (4) and possibly the U-shaped, T-shaped, or I-shaped profiling of the blade (13) by cold forming,
f) further laser treatment, punching, and/or cutting devices (46, 47, 48), in particular a corresponding automatic machine for producing the outer contour (8) and separating the implant plate blanks (1),
g) at least one small part magazine (43) for externally produced receiving members (6), e.g., eyes, hooks, or tube sections,
h) welding, pressure welding, soldering, screwing, and/or riveting devices (60, 61, 62, 63, 64), in particular a corresponding automatic machine for attaching externally produced receiving members (6) for flexible fastener elements (29) onto the side of the implant plate blank (1) facing away from the bone (4),
i) a cold working stretching device (65), in particular a corresponding automatic machine for generating weakened material areas (30) extending transversely to the longitudinal axis and sharpening the free end of the blade (13), but also converting the strip material (42) into a flat, trough-shaped profile which essentially no longer includes the width of the blade (13),
j) deburring, rounding, sandblasting, and/or cleaning devices (53, 54, 55, 56), in particular a corresponding automatic machine for removing sharp edge areas (8, 9, 11) on the implant plates (1),
k) a coating device (57) for surface finishing, in particular galvanic, electrochemical, and vacuum-technology coating devices, and
l) a sterilizer (58) and/or sterile packaging device (59), in particular a corresponding automatic machine, are used,
either the features d) and e) or g) and h) having to be present.

## Revendications

1. Plaque à implanter pourvue de perçages pour des vis à os, pour la stabilisation d'une fracture d'une tête humérale et/ou d'une fracture de l'humérus proximal, comprenant une plaque à implanter (1) en forme de cuillère, une partie côté tête (2) et une partie côté queue (3), chacune avec au moins un perçage (5) et/ou un perçage (5) en forme de trou oblong,
**caractérisée en ce que**
au moins une partie réceptrice en relief (6) pour un élément de fixation flexible (29) est disposée sur le côté de la partie côté tête (2) de la plaque à implanter (1) qui est opposé à l'os (4), sur l'arête extérieure ou encore le contour extérieur (8) de celle-ci,
chaque partie réceptrice (6) possède un évidement (7) s'étendant essentiellement parallèlement à l'arête extérieure ou encore au contour extérieur (8) et au côté de la partie côté tête (2) de la plaque à implanter (1) qui est opposé à l'os (4), pour enfiler, faire passer et contracter l'élément de fixation flexible (29).

2. Plaque à implanter selon la revendication 1, **caractérisée en ce que** le bord extérieur (9) de l'évidement (7) dans la partie réceptrice (6) pour l'élément de fixation flexible (29) est brisé, arrondi et/ou lissé du côté d'entrée et du côté de sortie.

3. Plaque à implanter selon les revendications 1 et 2, **caractérisée en ce que** la plaque à implanter (1) présente essentiellement la même épaisseur de matériau dans la partie côté tête (2) et dans la partie côté queue (3),
la partie côté tête (2) de la plaque à implanter (1) est élargie en forme de cuillère tandis que la partie côté queue (3) est réalisée plus étroite, et
chaque partie réceptrice (6) pour l'élément de fixation flexible (29) est disposée espacée sur l'arête extérieure ou encore le contour extérieur (8) de la partie côté tête (2).

4. Plaque à implanter selon les revendications 1 à 3, **caractérisée en ce que** l'évidement (7) de la partie réceptrice (6) est réalisé sous forme de perçage ou en forme d'oeillet, de tube ou de crochet rond.

5. Plaque à implanter selon les revendications 1 à 4, **caractérisée en ce que** les parties réceptrices (6) sont réalisées à partir de matériau en bande (42) par traitement au laser, découpage, coupe, emboutissage, pliage et roulage, et
les évidements (7) sont réalisés par perçage, découpage, traitement au laser, emboutissage, pliage et roulage.

6. Plaque à implanter selon les revendications 1 à 5, **caractérisée en ce que** les parties réceptrices (6) consistent en des talons (10) dotés de perçages (14), des parties réceptrices tubulaires (16) ou des crochets ronds (25) avec ou sans base (12), préfabriqué(e)s en externe, et
les parties réceptrices (6) sont soudées, soudées par pression, brasées, vissées ou rivetées sur les emplacements d'espacement prédéfinis (19), proches d'un bord, du matériau en bande (42).

7. Plaque à implanter selon les revendications 1 à 6, **caractérisée en ce que** tous les bords et arêtes (8, 9, 11) qui entrent en contact avec l'élément de fixation flexible (29) et le tissu humain sont brisés, arrondis et/ou lissés.

8. Plaque à implanter selon les revendications 1 à 7, **caractérisée en ce que** la partie côté tête (2) de la plaque à implanter (1) possède une lame (13) disposée dans le prolongement de l'axe longitudinal A-A et dotée d'une arête vive à une extrémité.

9. Plaque à implanter selon la revendication 8, **caractérisée en ce que** la lame (13) présente au moins un perçage (15) doté d'au moins un pas de vis respectif, dans lequel peuvent être vissées les vis de tête humérale partant de la partie côté tête (2) ou partie de tête (2) de la plaque à implanter (1)

10. Procédé de fabrication de plaques à implanter pour l'humérus,
**caractérisé en ce que**
une plaque à implanter en forme de cuillère, qui est dotée de perçages et/ou de trous oblongs pour des vis à os, est pourvue d'au moins une partie réceptrice pour un élément de fixation flexible sur le côté de la plaque à implanter qui est opposé à l'os, et
la partie réceptrice est dotée d'au moins un évidement qui s'étend essentiellement parallèlement à la face supérieure et à la face inférieure ainsi que parallèlement à l'arête extérieure ou encore au contour extérieur de la plaque à implanter.

11. Procédé selon la revendication 10, **caractérisé en ce que**
on met à disposition, mis à longueur ou sous forme de bobine, un matériau métallique en bande compatible avec le corps humain, comme de l'acier pour implants, du titane ou des alliages de titane.
le matériau en bande est usiné de manière commandée par programme dans un dispositif de perçage, de traitement au laser et/ou de découpage afin de réaliser les évidements de relativement grand diamètre qui sont nécessaires pour les vis à os,
simultanément ou consécutivement, le matériau en bande est usiné de manière commandée par programme dans un dispositif de perçage, de traitement au laser et/ou de découpage afin de réaliser les évidements de relativement petit diamètre qui sont nécessaires pour des éléments de fixation flexibles,
simultanément ou consécutivement, les coupes de façonnage proches du bord, telles que coupes parallèles, coupes biaises, découpes partielles et analogues, qui sont nécessaires pour la formation des parties réceptrices et éventuellement d'une lame sont réalisées dans un dispositif de découpage, de traitement au laser, de coupe ou analogue,
les régions du matériau en bande préparées pour la formation des parties réceptrices proches du bord sont embouties, pliées, roulées, repliées, et autres traitements analogues, près du bord en direction du côté de l'implant qui est opposé à l'os,
les évidements des parties réceptrices, formés par perçage, découpage, traitement au laser, emboutissage, pliage, roulage, repliage et analogues, sont disposés en s'étendant essentiellement parallèlement à la face supérieure et à la face inférieure ainsi qu'essentiellement parallèlement à l'arête extérieure ou encore au contour extérieur de la plaque à implanter, et
tous les bords et arêtes de la plaque à implanter sont brisés et/ou arrondis ainsi que lissés.

12. Procédé selon les revendications 10 et 11, **caractérisé en ce que**
une plaque à implanter en forme de cuillère, qui est dotée de perçages pour des vis à os, est pourvue d'au moins une partie réceptrice à évidement intégré, formée en externe, pour un élément de fixation flexible, sur le côté de la plaque à implanter qui est opposé à l'os, et
l'évidement dans la partie réceptrice est disposé essentiellement parallèlement à la face supérieure et à la face inférieure ainsi que parallèlement à l'arête extérieure ou encore au contour extérieur de la plaque à implanter.

13. Installation pour la fabrication de plaques à implanter selon la revendication 9 ou pour la mise en oeuvre d'un procédé selon la revendication 11 ou 12, **caractérisée en ce qu'**on utilise
a) au moins un magasin d'empilage (40) ou au moins un dispositif de dévidage (41) pour la fourniture et l'alimentation de matériau métallique en bande (32),
b) un laminoir (44) pour profiler au moins d'un côté la surface du matériau en bande (42), par exemple pour la production de nopes, de rainures ou losanges interrompus et de profils analogues sur le côté tourné vers l'os (4),
c) un dispositif (45, 46, 47) de perçage, de traitement au laser et/ou de découpage, notamment un automate correspondant, pour la réalisation d'évidements (7), de pré-trous ou de perçages (14, 23) et analogues,
d) un dispositif (46, 47, 48) de traitement au laser, de découpage et/ou de coupe, notamment un automate correspondant, pour la préparation du matériau en bande (42) pour la formation de parties réceptrices en relief (6) sur le côté de la plaque à implanter (1) qui est opposé à l'os (4), et éventuellement d'une lame (13) partant de la partie en forme de cuillère (2) de l'ébauche (1) de plaque à implanter,
e) des dispositifs (49, 50, 51) d'emboutissage, de pliage et de roulage, notamment un automate correspondant, pour le façonnage des parties réceptrices en relief (6) sur le côté de l'ébauche (1) de plaque à implanter qui est opposé à l'os (4), et éventuellement pour le profilage en forme de U, de T ou de 1 de la lame (13) par déformation à froid,
f) d'autres dispositifs (46, 47, 48) de traitement au laser, de découpage et/ou de coupe, notamment un automate correspondant, pour la réalisation du contour extérieur (8) et l'individualisation des ébauches (1) de plaque à implanter,
g) au moins un magasin (43) de petites pièces pour des parties réceptrices (6) fabriquées en externe, par exemple des oeillets, des crochets ou des tronçons de tube,
h) des dispositifs (60, 61, 62, 63, 64) de soudage, soudage par pression, brasage, vissage et/ou rivetage, notamment un automate correspondant, pour la fixation de parties réceptrices (6) fabriquées en externe, pour des éléments de fixation flexibles (29), sur le côté de l'ébauche (1) de plaque à implanter qui est opposé à l'os (4),
i) un dispositif (65) d'étirage à froid, notamment un automate correspondant, pour produire des régions (30) d'affaiblissement de matériau s'étendant transversalement à l'axe longitudinal et pour affiler l'extrémité libre de la lame (13), ainsi que pour transformer le matériau en bande (42) en un profil plat en forme de gouttière, n'englobant pour l'essentiel plus la largeur de la lame (13),
j) des dispositifs (53, 54, 55, 56) d'ébarbage, d'arrondi, de sablage et/ou de nettoyage, notamment un automate correspondant, pour supprimer les régions d'arêtes vives (8, 9, 11) sur les plaques à implanter (1),
k) un dispositif de revêtement (57) pour le traitement de surface, notamment des dispositifs de revêtement galvaniques, électrochimiques et sous vide, et
l) un stérilisateur (58) et/ou un dispositif (59) de conditionnement stérile, notamment un automate correspondant,
sachant que les caractéristiques d) et e) ou g) et h), au choix, doivent être présentes.
